# FASCICULE DE BREVET EUROPEEN

(11) **EP 2 421 885 B1**
(45) Date de publication et mention de la délivrance du brevet: **25.02.2015**
(21) Numéro de dépôt: 10718243.8
(22) Date de dépôt: 22.04.2010
(51) Int. Cl.: C07K 5/093, C07K 5/103, A61K 8/64, C07K 7/06, C07K 14/395, C07K 14/415, C07K 5/113, A61Q 19/02

(54) **HYDROLYSATS PEPTIDIQUES ECLAIRCISSANTS ACTIVATEURS DU PROTEASOME ET COMPOSITIONS LES CONTENANT**
BLEICHENDES PEPTIDHYDROLYSAT ALS PROTEASOMAKTIVATOR UND ZUSAMMENSETZUNGEN, DIE DIESES ENTHALTEN
BLEACHING PEPTIDE HYDROLYSATE PROTEASOME ACTIVATOR AND COMPOSITIONS CONTAINING SAME

(30) Priorité: 23.04.2009 FR 0901979
(43) Date de publication de la demande: 29.02.2012
(73) Titulaire: Isp Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000325
(87) Numéro de publication internationale: WO 2010/122245

(56) Documents cités:
- WO-A1-2005/107697
- WO-A1-2008/009709
- WO-A2-02/080876
- WO-A2-2008/015343
- FR-A1- 2 904 552
- FR-A1- 2 915 378
- FR-A1- 2 915 379
- FR-A1- 2 915 380
- FR-A1- 2 915 381
- FR-A1- 2 915 382
- FR-A1- 2 915 383
- FR-A1- 2 915 384

### SEQUENCE LISTING

<110> ISP Investments INC.
<120> HYDROLYSATS PEPTIDIQUES ECLAIRCISSANTS ACTIVATEURS DU PROTEASOME ET COMPOSITIONS LES CONTENANT
<130> Bv PCT 09-127
<150> FR 0901979
   <151> 2009-04-23
<160> 5
<170> PatentIn version 3.3
<210> 1
   <211> 5
   <212> PRT
   <213> Plant OR Saccharomyces cerevisiae
<400> 1
   Arg Asp Cys Arg Arg
   1               5
<210> 2
   <211> 5
   <212> PRT
   <213> plant OR saccharomyces cerevisiae
<400> 2
   Asn Asp Cys Arg Lys
   1               5
<210> 3
   <211> 4
   <212> PRT
   <213> Plant OR saccharomyces cerevisiae
<400> 3
   Asp Cys Arg His
   1
<210> 4
   <211> 4
   <212> PRT
   <213> Plant OR saccharomyces cerevisiae
<400> 4
   Val Asp Cys Arg
   1
<210> 5
   <211> 3
   <212> PRT
   <213> Plant OR saccharomyces cerevisiae
<400> 5
   Asp Cys Arg
   1

## Description

La présente invention se rapporte au domaine des actifs dépigmentants et de leurs utilisations en cosmétique notamment. Plus particulièrement, la présente invention se rapporte à l'utilisation d'un hydrolysat peptidique enrichi en peptides bioactifs, ledit hydrolysat étant activateur du protéasome, en cosmétique en tant qu'agent éclaircissant, dépigmentant et/ou blanchissant. Ledit hydrolysat permet en outre de prévenir et/ou traiter les signes cutanés d'ordre hyperpigmentaires dus au photo-vieillissement, ainsi que les taches d'hyperpigmentation d'origines diverses.

Chez l'humain, la couleur des cheveux et de la peau est liée à des facteurs individuels (origine ethnique, sexe, âge, etc.) et à des facteurs environnementaux (notamment les saisons de l'année, la zone d'habitation, etc.). Elle est principalement déterminée par la nature et la concentration de mélanine produite par les mélanocytes. La mélanine a la propriété de protéger les cellules cutanées des effets délétères des rayonnements UV et de ralentir le photo-vieillissement cutané. Les mélanocytes sont les cellules spécialisées qui, par l'intermédiaire d'organites particuliers, les mélanosomes, synthétisent la mélanine. La synthèse de la mélanine ou mélanogénèse est un processus complexe dont les mécanismes précis ne sont pas encore élucidés et qui fait intervenir schématiquement les étapes suivantes :

Tyrosine → Dopa → Dopaquinone → Dopachrome → Mélanine.

Cette mélanine joue en effet un rôle fondamental dans la détermination de la couleur de la peau. Elle est synthétisée par de larges cellules dendritiques : les mélanocytes, cellules localisées dans la couche basale de l'épiderme. La mélanine existe sous deux formes différentes : phaeomélanine, pigment de couleur jaune, et l'eumélanine de couleur noire. Ce sont les différentes proportions et la taille de ces pigments, sans oublier les caroténoïdes et la micro-circulation sanguine, qui donnent à la peau sa grande diversité de couleur.

La production de mélanine, ainsi que son transport, sont régulés par différents facteurs tels que, par exemple, le rayonnement UV, les hormones ou les produits chimiques. Ainsi, une augmentation de l'exposition aux rayonnements UV provoque la synthèse de pigments et résulte en un assombrissement de la peau. Des perturbations de cette pigmentation, plus ou moins bénignes, peuvent apparaître. Elles se manifestent, par exemple, par des taches de rousseur, des grains de beauté, des taches diffuses telles que les taches de grossesse, le chloasma, le mélasma, ainsi que par d'autres désordres hyperpigmentaires comme par exemple le lentigo. D'autres taches d'hyperpigmentation peuvent être dues par exemple à une mauvaise cicatrisation notamment chez les individus de phénotype foncé, ou encore elles peuvent être dues à l'utilisation de médicaments photo-sensibilisants etc. Mais le vieillissement peut lui aussi moduler la pigmentation cutanée. Ainsi, certaines personnes peuvent voir apparaître des taches sur la peau, plus ou moins foncées ou colorées, conférant des zones de colorations hétérogènes formant des taches de sénescence ou encore des éphélides. Enfin, dans certaines populations de type asiatique ou encore africaine, des soins cosmétiques éclaircissants sont recherchés afin d'obtenir un teint clair et uniforme.

L'utilisation d'inhibiteur ou de régulateur de la synthèse de mélanine, ainsi que de tout autre produit dépigmentant et/ou blanchissant, est donc particulièrement intéressant en cosmétologie et/ou en dermatologie. Cette utilisation est non seulement intéressante lorsqu'une véritable dépigmentation de la peau est recherchée, comme dans le cas de blanchiment de peau fortement pigmentée ou encore lors d'inhibition de zones cutanées hyperpigmentées résultant en un aspect inesthétique de la peau ; elle l'est également dans certaines applications visant à éclaircir le teint, à donner de la luminosité à la peau ou encore à donner de l'éclat aux tissus de surface.

Jusqu'à ce jour, de nombreuses molécules ont été proposées avec plus ou moins d'efficacité. Parmi ces molécules, on peut citer des dérivés du phénol tels que l'hydroquinone et le résorcinol qui inhibent une série de réaction de la conversion de la L- tyrosine en mélanine par inhibition de l'activité de la tyrosinase (Takano, 1984). On peut citer aussi l'acide L-ascorbique et ses dérivés, le magnésium ascorbyl acétate, l'acide kojique ou encore l'acide lactique.

Mais la plupart des produits actuellement sur le marché sont toxiques et/ou ne présentent pas une efficacité suffisante. Par exemple, l'hydroquinone est irritante et cytotoxique pour le mélanocyte. L'acide kojique par exemple n'est pas stable en solution, etc. Il existe donc un besoin pour un nouvel agent blanchissant qui ne présenterait pas les inconvénients de ceux existants mais qui serait tout aussi efficace. C'est ainsi que d'autres voies ont été explorées afin de trouver un nouvel agent dépigmentant ayant une action à la fois sur l'enzyme tyrosinase et sur la mélanine.

De manière surprenante, la Demanderesse a découvert qu'un hydrolysat peptidique enrichi en peptides bioactifs, ledit hydrolysat étant activateur du protéasome pouvait être utilisé en tant qu'agent blanchissant et présentait une bonne activité dépigmentante sans pour autant être toxique.

La voie ubiquitine-protéasome joue un rôle fondamental dans un très grand nombre de processus biologiques. En effet, les mécanismes de dégradation des protéines par le protéasome sont impliqués dans des mécanismes cellulaires importants tels que la réparation de l'ADN, le contrôle de l'expression des gènes, la régulation de la progression du cycle cellulaire, le contrôle de la qualité des protéines néosynthétisées, l'apoptose ou la réponse immunitaire (Glickman and Ciechanover, 2002).

Le protéasome présent dans les cellules humaines est un complexe multi protéique de très grande taille présent dans le cytoplasme et le noyau. Les formes purifiées du protéasome comprennent 2 grandes sous-unités ; d'une part, un coeur protéolytique appelé protéasome 2OS et, d'autre part, un complexe régulateur 19S qui se lie à chacune des deux extrémités du protéasome 2OS (Coux et al., 1996 ; Glickman et Coux, 2001). Le protéasome 2OS est une particule en forme de cylindre creux, composée de 28 sous-unités alpha et béta, distribuées en 4 anneaux heptamériques. Les activités peptidases sont présentes à la surface interne du cylindre et s'influencent de manière allostérique. Trois activités protéolytiques (« trypsine, chymotrypsine et caspase-like ») ont été associées au protéasome 2OS et concourent à la destruction des protéines en peptides inactifs de 3 à 20 acides aminés. Outre le protéasome 2OS, le protéasome 26S comprend le complexe régulateur 19S de 0,7 MDa constitué de 20 sous-unités environ. Des études récentes d'immunopurification ont montré que d'autres protéines pouvaient être associées au protéasome 20S et 19S (par exemple le complexe régulateur 11 S).

Au vu de la diversité des processus cellulaires contrôlés via la dégradation des protéines, il n'est pas surprenant de constater que des altérations de la voie ubiquitine-protéasome sont à l'origine, ou étroitement liées à plusieurs maladies génétiques et à de nombreuses pathologies humaines telles que les cancers colorectaux, les lymphomes, les syndromes inflammatoires, les maladies neuro-dégénératives telles que Parkinson ou Alzheimer...

De nombreux travaux ont été menés ces dernières années sur le rôle du protéasome dans le vieillissement de la peau. Une des voies explorées dernièrement se tournait vers le protéasome et son action sur la dégradation des protéines impliquées dans la mélanogénèse. Des expériences ont montrées que la dégradation de l'enzyme tyrosinase par le protéasome était activée par un extrait d'algues sur des mélanocytes humains (Nizard et al. Antioxid. Redox Signal ; 2006, 1-2 : 136-143).

Une composition comprenant un extrait de silybine, de Bletilla striata et d'Iris sanguinea capable d'augmenter l'activité du protéasome et permettant d'avoir un effet sur la pigmentation de la peau a été divulguée dans une demande de brevet (US 12/088.919)

Le document FR2915383 décrit une composition comprenant un hydrolysat de riz pour protéger la peau, des dommages oxydatifs causés par les rayonnements UV.

C'est ainsi que la Demanderesse a découvert qu'un hydrolysat peptidique enrichi en peptides bioactifs était capable d'activer le protéasome et pouvait ainsi dépigmenter, éclaircir ou encore blanchir la peau et les phanères.

Le document WO2008015343 décrit l'utilisation d'un extrait de levure pour le bronzage et/ou brunissage artificiels de la peau.

Par conséquent la présente invention a pour premier objet l'utilisation d'une composition comprenant un hydrolysat peptidique activateur du protéasome, issu de l'hydrolyse de levures du genre *Saccharomyces,* et plus particulièrement de l'espèce *Saccharomyces cerevisiae* et enrichi en peptides bioactifs de poids moléculaire inférieur à 6 kDa, comprenant de 3 à 5 acides aminés, chaque peptide bioactif comprenant au moins un résidu acide aspartique, un résidu cystéine et un résidu arginine et un milieu cosmétiquement acceptable, pour augmenter l'activité du protéasome et améliorer la dégradation par le protéasome des protéines endommagées impliquées dans la mélanogénèse et ainsi dépigmenter, la peau et/ou les phanères, et prévenir et/ou traiter les taches d'hyperpigmentation.

Enfin, la présente invention a pour second objet un procédé de traitement cosmétique de la peau ou des phanères à traiter à l'aide de la composition comprenant ledit hydrolysat peptidique issu de l'hydrolyse de levures du genre *Saccharomyces,* et plus particulièrement de l'espèce *Saccharomyces cerevisiae* et enrichi pour dépigmenter, éclaircir et/ou blanchir la peau ou les phanères.
La figure 1 représente un exemple de chromatogramme obtenu par CLHP, avec mise en évidence du pic correspondant au peptide bioactif dans un hydrolysat de maïs.
La figure 2 représente un exemple de chromatogramme obtenu par CLHP, avec mise en évidence du pic correspondant au peptide bioactif dans un hydrolysat de pois.
La figure 3 représente un exemple de chromatogramme obtenu par CLHP, avec mise en évidence du pic correspondant au peptide bioactif dans un hydrolysat de riz.
La figure 4 représente un exemple de chromatogramme obtenu par CLHP, avec mise en évidence du pic correspondant au peptide bioactif dans un hydrolysat de Saccharomyces cerevisiae.

La présente invention a pour objet premier l'utilisation d'une composition comprenant un hydrolysat peptidique activateur du protéasome, issu de l'hydrolyse de levures du genre *Saccharomyces,* et plus particulièrement de l'espèce *Saccharomyces cerevisiae* et enrichi en peptides bioactifs de poids moléculaire inférieur à 6 kDa, comprenant de 3 à 5 acides aminés, chaque peptide bioactif comprenant au moins un résidu acide aspartique, un résidu cystéine et un résidu arginine et un milieu cosmétiquement acceptable, pour augmenter l'activité du protéasome et améliorer la dégradation par le protéasome des protéines endommagées impliquées dans la mélanogénèse.

On entend par « hydrolysat peptidique », un mélange de composés majoritairement représentés par des peptides ou des oligopeptides.

Par « peptides bioactifs » on entend un fragment de protéine composé par l'enchaînement d'au moins 3 acides aminés liés entre eux par des liaisons peptidiques modifiées ou non et qui présentent une activité en tant qu'activateur du protéasome. Présents sous forme inactive dans les protéines, ils deviennent actifs après hydrolyse de ces dernières.

Ledit hydrolysat peptidique enrichi utilisé selon l'invention est caractérisé en ce qu'il est activateur du protéasome.

On entend par hydrolysat peptidique (et/ou peptides bioactifs) « activateur du protéasome», tout hydrolysat peptidique, ou peptide ou dérivé biologiquement actif capable d'augmenter l'activité du protéasome, soit par augmentation de la synthèse protéique des sous-unités du protéasome (par modulation directe ou indirecte de l'expression génique), soit par d'autres processus biologiques tels que la stabilisation des sous-unités constituant le protéasome ou encore la stabilisation des transcrits d'ARN messager.

L'hydrolysat peptidique enrichi utilisé selon l'invention est caractérisé en ce qu'il permet d'activer la dégradation par le protéasome des protéines endommagées. Par « protéines endommagées » on entend des protéines ayant subi des réactions d'oxydation dues aux espèces réactives de l'oxygène (radicaux libres), des protéines glyquées ou conjuguées avec des produits issus de la péroxydation lipidique etc.

Préférentiellement, l'hydrolysat peptidique utilisé est enrichi en peptides bioactifs de formule générale (I) :

X₁ - [Asp, Cys, Arg] - X₂

Dans laquelle,
X₁ est une asparagine, une lysine, un aspartate, une valine, une arginine, ou est absent
X₂ est une histidine, une lysine, une arginine, ou est absent

Préférentiellement, l'hydrolysat peptidique utilisé est riche en peptides bioactifs de formule suivante:
(SEQ ID n°1) Arg - Asp -Cys -Arg - Arg
(SEQ ID n°2) Asn - Asp -Cys-Arg-Lys
(SEQ ID n°3) Asp -Cys-Arg-His
(SEQ ID n°4) Val-Asp -Cys -Arg
(SEQ ID n°5) Asp -Cys -Arg

En effet, ces peptides ont été identifiés comme étant particulièrement actifs en tant qu'activateurs du protéasome et présentent donc un intérêt particulier en tant qu'agent éclaircissant.

L'hydrolysat peptidique enrichi utilisé est issu de l'hydrolyse de levures du genre Saccharomyces, et plus particulièrement de l'espèce *Saccharomyces cerevisiae.*

L'hydrolysat peptidique enrichi peut être obtenu à partir de diverses sources de protéines, qu'elles soient d'origine animale ou végétale. Selon un autre mode de réalisation, l'hydrolysat peptidique enrichi est issu de l'hydrolyse de plantes choisies parmi le maïs *(Zea mayz L.),* le pois *(Pisum sativum),* ou le riz *(Oryza sativa L.).* De préférence, les plantes utilisées ne sont pas soumises à une fermentation préalable.

Ainsi, on peut utiliser les graines d'une des nombreuses plantes du genre Zea et préférentiellement l'espèce *Zea mays L.* Le matériel végétal utilisé sera le grain et préférentiellement le grain débarrassé de son enveloppe par une étape de décorticage.

On peut également utiliser l'une des nombreuses plantes de la famille des pois (Fabacées). On utilisera, par exemple, les plantes de l'espèce des pois *Pisum sativum L.* Le terme pois désigne en outre la graine, elle-même riche en protéines (25 %).

Les plantes de la famille du riz (Poacées), notamment celles du genre Oryza et plus préférentiellement l'espèce *Oryza sativa L* peuvent être utilisées pour réaliser l'hydrolysat. Le matériel végétal utilisé sera le grain et préférentiellement le grain débarrassé de son enveloppe par une étape de décorticage.

Toute méthode d'extraction ou de purification connue de l'homme du métier peut être utilisée afin de préparer l'hydrolysat utilisé selon l'invention.

Lorsqu'il s'agit de levures, dans une première étape, celles-ci sont mises en culture de manière classique dans un milieu adapté à leur développement, de préférence en présence de lactose. Elles sont récoltées par centrifugation puis mises en suspension dans une solution tampon, préférentiellement un tampon phosphate. Dans une seconde étape, ces cellules sont éclatées à l'aide d'une presse de French ou à l'aide d'un broyeur à bille, la majorité des composants membranaires insolubles étant écartés par centrifugation ou par filtration.

Dans une première étape, selon un autre mode de réalisation, les graines, ou une partie spécifique de la plante (feuilles, tubercules, racines, etc.) sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

On réalise ensuite l'extraction des protéines suivant le procédé classique (Osborne, 1924) modifié ; le broyat de plante ou le lysat de levures est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromélaïne, ficine) et de micro-organismes (Aspergillus, Rhizopus, Bacillus, etc.). Les conditions d'hydrolyses sont choisies pour favoriser l'enrichissement en peptides bioactifs.

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après filtration, permettant d'éliminer les enzymes et les polymères, un premier filtrat est obtenu.

L'hydrolysat obtenu à ce stade peut être encore purifié afin de sélectionner les fractions de bas poids moléculaires, préférentiellement inférieures à 6 kDa, ainsi que les peptides générés selon leur nature. La purification s'effectue avantageusement par des étapes d'ultrafiltrations successives à travers des filtres de porosité décroissante, en conservant les filtrats à chaque étape et/ ou par une méthode de type chromatographique, afin d'enrichir spécifiquement l'hydrolysat en peptides bioactifs.

On procède à une phase de dilution dans de l'eau ou dans tout mélange contenant de l'eau, puis stérilisation par ultrafiltration afin d'obtenir un hydrolysat peptidique enrichi caractérisé par une teneur en protéines de 0,5 à 5,5 g/l. Cet hydrolysat peptidique enrichi correspond à la forme la plus purifiée du principe actif utilisé selon l'invention.

L'hydrolysat peptidique obtenu utilisé selon l'invention est analysé qualitativement et quantitativement en chromatographie liquide haute pression (CLHP), qui permet d'analyser les protéines ayant des poids moléculaires de 0,2 à 25 kDa (selon un gradient de solvants approprié). Les différentes fractions peptidiques qui ont pu être isolées sont ensuite analysées pour leur efficacité biologique. Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs.

Finalement, l'hydrolysat peptidique enrichi obtenu est composé de peptides de poids moléculaire inférieur à 6 kDa, et est enrichi en peptides bioactifs de 3 à 5 acides aminés comprenant au moins un résidu acide aspartique, un résidu cystéine et un résidu arginine.

De manière préférée, les compositions utilisées selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit hydrolysat peptidique est présent dans la composition en une quantité représentant de 0,0001 % à 20 % du poids total de la composition, et préférentiellement en une quantité représentant de 0,05 % à 5 % du poids total de la composition.

Dans les compositions utilisées selon l'invention, l'hydrolysat peptidique enrichi en peptides bioactifs est solubilisé dans un ou plusieurs solvants comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, l'hydrolysat peptidique utilisé selon l'invention est solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydroalcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotion, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition utilisée selon l'invention contient en outre, au moins un autre principe actif favorisant l'action dudit hydrolysat peptidique enrichi. On peut citer, de manière non limitative, les classes d'ingrédients présentant une activité dans le domaine des agents éclaircissants tels que des agents desquamants; des agents apaisants, des agents photo-protecteurs organiques ou inorganiques, des agents hydratants; d'autres agents dépigmentants, des inhibiteurs de tyrosinase; des agents stimulant la synthèse de macromolécules dermiques ou épidermiques et/ou empêchant leur dégradation; des agents stimulant la prolifération des fibroblastes et/ou des kératinocytes ou stimulant la différenciation des kératinocytes; des agents agissant sur le métabolisme énergétique des cellules; d'autres peptides dépigmentants, des hydrolysats végétaux, des agents anti-âge, ainsi que leurs mélanges. En outre, des additifs tels que des agents épaississants, émulsifiants, humectants, émollients, des parfums, des antioxydants, des agents filmogènes, chélatants, séquestrants, conditionnants... peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition utilisée selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids, par rapport au poids total de la composition.

Enfin, la composition telle que décrite permet d'augmenter l'activité du protéasome et d'améliorer la dégradation par le protéasome des protéines endommagées impliquées dans la mélanogènese.

L'invention se rapporte à l'utilisation d'une composition cosmétique comprenant ledit hydrolysat peptidique enrichi et un milieu cosmétiquement acceptable pour dépigmenter, éclaircir et/ou blanchir la peau.

La composition utilisée selon l'invention permet aussi de prévenir et/ou traiter les taches d'hyperpigmentation telles que le mélasma, le chloasma, le lentigo actinique, les lentigos solaires, les éphélides, les taches d'hyperpigmentation accidentelles, les taches d'hyperpigmentation post- cicatricielles.

L'invention se rapporte à l'utilisation d'une composition comprenant ledit composé peptidique pour traiter et/ou prévenir les signes cutanés d'ordre hyperpigmentaire dus au photo-vieillissement. Par « photo-vieillissement » on entend le vieillissement prématuré de la peau causé par l'exposition prolongée et cumulative au soleil.

Un dernier objet de la présente invention se rapporte à un procédé de traitement cosmétique caractérisé en ce que l'on applique topiquement sur la peau ou les phanères à traiter, une composition comprenant une quantité efficace d'hydrolysat peptidique utilisé selon l'invention pour dépigmenter, éclaircir et/ou blanchir la peau ou les phanères. Par ailleurs, ce procédé de traitement cosmétique est aussi destiné à prévenir et/ou traiter les signes cutanés d'ordre hyperpigmentaire dus au photovieillissement.

Les exemples suivants décrivent et démontrent l'efficacité de composés peptidiques utilisés tels que décrits selon l'invention.

### Exemple 1 : Préparation d'un hydrolysat peptidique à partir de tourteaux de maïs (Zea mays L.) (exemple de référence)

Le tourteau de maïs (*Zea mays L.)* est mis en solution dans 10 volumes d'eau en présence de 2 % de POLYCLAR® 10 (polyvinylpolypyrrolidone - PVPP - insoluble). Le mélange est ajusté à un pH compris entre 6 et 8 avec une solution aqueuse de soude 1 M.

Après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de maïs est récupérée. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification de l'hydrolysat brut commence par des filtrations successives à l'aide de filtres-plaques Seitz-Orion de porosité décroissante (jusqu'à 0.2 µm) afin d'obtenir une solution jaune, brillante et limpide, qualifiée d'hydrolysat 1.

A cette étape, l'hydrolysat 1 de maïs est caractérisé par un extrait sec titrant de 20 à 30 g/kg, un taux de protéines de 20 à 25 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de maïs est chauffé à 70°C pendant 10 minutes dans des conditions réductrices dénaturantes dans un tampon de préparation d'échantillon NuPAGE® LDS. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe des protéines de poids moléculaire inférieur à 6 kDa.

L'hydrolysat 1 est ensuite purifié en éliminant les protéines de haut poids moléculaire par ultrafiltration à l'aide de la cassette Pellicon® 2 Biomax 5 kDa afin de ne conserver que les composés de nature peptidique inférieurs à 5 kDa.

Après cette purification finale, on procède à une phase de dilution afin d'obtenir un hydrolysat peptidique caractérisé par un taux de protéines compris entre 3,5 et 5,5 g/l. Cet hydrolysat peptidique enrichi en peptides bioactifs correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa dans les conditions suivantes:
- Gradient Méthanol
- Colonne Uptisphere OPB 125 x 3 mm
- Solvant A: eau grade HPLC contenant 0.1% d'acide heptafluorobutyrique (HFBA)
- Solvant B: méthanol grade HPLC contenant 0.1% d'acide heptafluorobutyrique (HFBA)
- Gradient: 100% à 15% solvant A en 35 min

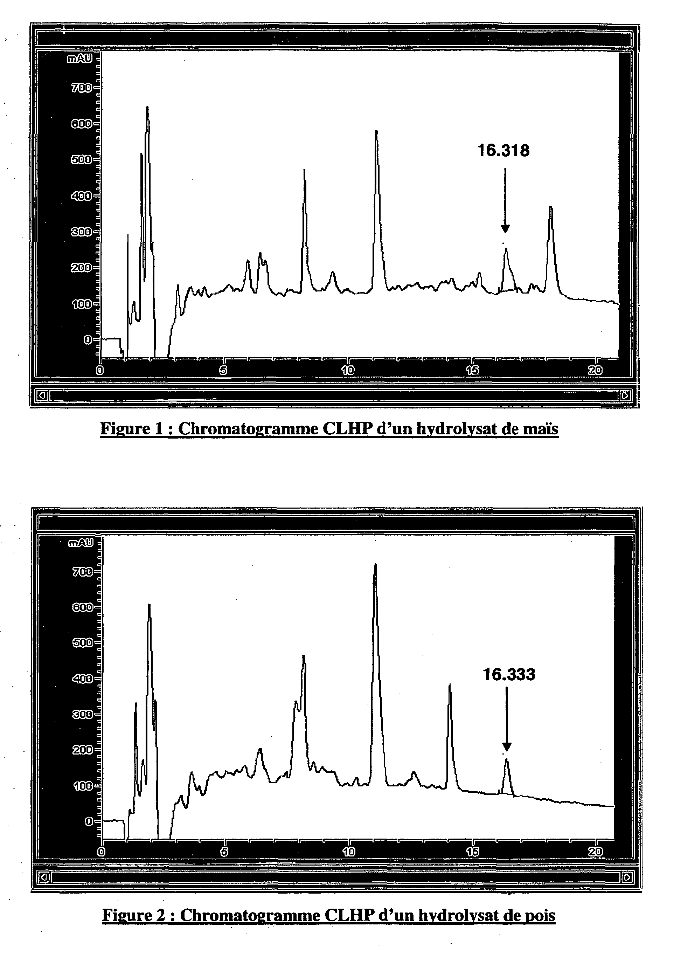

Un exemple de chromatogramme obtenu par CLHP (chromatographie en phase liquide à haute pression), avec mise en évidence du pic correspondant aux peptides bioactifs est donné à la figure 1.

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs.

### Exemple 2: Préparation d'un hydrolysat peptidique enrichi en peptides bioactifs à partir de pois (Pisum sativum L.) (exemple de référence)

L'hydrolysat peptidique est obtenu à partir d'un extrait de plantes de l'espèce *Pisum sativum L.* Bien entendu l'extrait peut être préparé à partir de plantes d'au moins l'une quelconque des nombreuses variétés et espèces appartenant au genre Pisum.

Dans une première étape, 1 kg de pois décortiqués sont délipidés par l'action d'un solvant organique : l'hexane.

La farine de pois ainsi obtenue est mise en solution dans 10 volumes d'eau en présence de 2 % de POLYCLAR® 10 (polyvinylpolypyrrolidone - PVPP - insoluble). Le mélange est ajusté à un pH compris entre 6 et 7 avec une solution aqueuse de soude 1 M.

Après ajustement du pH, il est rajouté 2 % de flavourzym® dans le milieu réactionnel. L'hydrolyse est obtenue après 2 heures d'agitation à 50°C. On procède à l'inactivation de l'enzyme en chauffant la solution à 80°C pendant 2 heures. Le mélange réactionnel ainsi obtenu correspond à l'extrait de pois. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'hydrolysat de pois est caractérisé par un sec titrant de 70-80 g/kg, un taux de protéines de 55-65 g/l, un taux de sucres de 2-5 g/l et un taux de polyphénol de 1-3 g/l.

La nature protéique de cet hydrolysat est mise en évidence par électrophorèse sur gel de polyacrylamide. Pour cette analyse, on utilise les gels NuPAGE® Bis-Tris Pre-cast (Invitrogen). L'hydrolysat peptidique de pois est chauffé à 70°C pendant 10 minutes dans des conditions réductrices dénaturantes dans un tampon de préparation d'échantillon NuPAGE® LDS. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 2 grandes familles de protéines : la 1ère famille correspond à des protéines de poids moléculaire de 20 à 25 kDa et la dernière famille à des protéines de poids moléculaires inférieurs à 5kDa.

Cette solution est alors purifiée en éliminant les protéines de poids moléculaires supérieurs à 5 kDa à l'aide d'une filtration à flux tangentiel. Pour cela, l'hydrolysat de pois est pompé sous pression à travers un support Pellicon® équipé de cassette Pellicon® 2 Biomax 30 kDa. Ce 1er filtrat est récupéré pour être ensuite filtré à travers une autre cassette Pellicon® 2 Biomax 5 kDa. En fin de purification, on obtient un hydrolysat peptidique de pois jaune-beige, brillant et limpide. Il est caractérisé par un sec de 50 à 55 g/kg, une teneur en protéines de 50 à 52 g/l.

Cette solution est alors analysée en chromatographie liquide haute pression à l'aide d'un appareil HP1100 piloté par le logiciel ChemStation. La colonne utilisée lors de l'élution de l'extrait de pois est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont pu être isolées.

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs. Un exemple de chromatogramme obtenu par CLHP (chromatographie en phase liquide à haute pression), avec mise en évidence du pic correspondant aux peptides bioactifs est donné à la figure 2.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 3: Préparation d'un hydrolysat peptidique enrichi en peptides bioactifs à partir de levures Saccharomyces cerevisiae

L'hydrolysat peptidique peut être obtenu à partir d'un extrait de levures de l'espèce *Saccharomyces cerevisiae.* Les levures sont cultivées dans un milieu adapté à leur développement, de préférence en présence de lactose, puis centrifugées pour récupérer une biomasse. La biomasse de saccharomyces est mise en solution dans 10 volumes d'eau en présence de 2 % de polyvinylpyrrolidone (POLYCLAR® 10 - insoluble) et 0,2 % de charbon actif. Le mélange est ajusté à un pH compris entre 6 et 7,5 avec une solution aqueuse de soude 1 M.

Après ajustement du pH, il est rajouté 2 % de papaïne dans le milieu réactionnel. L'hydrolyse est obtenue après 2 heures d'agitation à 55°C. On procède à l'inactivation de l'enzyme en chauffant la solution à 80°C pendant 2 heures. Après centrifugation, le mélange réactionnel correspondant à l'extrait de saccharomyces est alors obtenu. Des conditions spécifiques d'hydrolyse sont choisies de façon à permettre un enrichissement en peptides bioactifs de 3 à 5 acides aminés comprenant au moins un résidu acide aspartique, un résidu cystéine et un résidu arginine.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'extrait de saccharomyces est caractérisé par un sec de 25 à 35 g/kg, un taux de protéines de 10 à 15 g/l et un taux de sucres de 5-10 g/l.

La nature protéique de cet extrait est mise en évidence par électrophorèse sur gel de polyacrylamide. Pour cette analyse, les gels NuPAGE® Bis-Tris Pre-cast (Invitrogen) sont utilisés. L'hydrolysat peptidique est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 3 grandes familles de protéines : la 1ère famille correspond à des protéines de poids moléculaire supérieur à 75 kDa, la 2ème famille à des protéines de 20 à 25 kDa et la dernière famille à des protéines de poids moléculaire inférieur à 5 kDa.

Cette solution est alors purifiée en éliminant les protéines de poids moléculaire supérieur à 5 kDa à l'aide de filtration à flux tangentiel. Pour cela, l'hydrolysat peptidique de saccharomyces est pompé sous pression à travers un support Pellicon® équipé de cassette Pellicon® 2 Biomax 50 kDa. Ce 1er filtrat est récupéré pour être ensuite filtré à travers une seconde cassette Pellicon® 2 Biomax 10 kDa. Il est alors récupéré un second filtrat qui est encore élué à travers une dernière cassette Pellicon® 2 Biomax 5 kDa. En fin de purification, on obtient un extrait végétal de saccharomyces beige, brillant et limpide. Il est caractérisé par un sec de 35 à 45 g/kg, une teneur en protéines de 30 à 40 g/l.

Cette solution est alors analysée en chromatographie liquide haute pression à l'aide d'un appareil HP1100 piloté par le logiciel ChemStation. La colonne utilisée lors de l'élution de l'hydrolysat de saccharomyces est une Nucleosil® 300-5 C4 MPN (125 x 4 mn). Cette colonne permet de chromatographier des protéines ayant des poids moléculaires de 0,2 à 25 kDa (selon un gradient de solvants approprié, identique à l'exemple 1). Dans ces conditions chromatographiques, plusieurs fractions peptidiques ont été isolées.

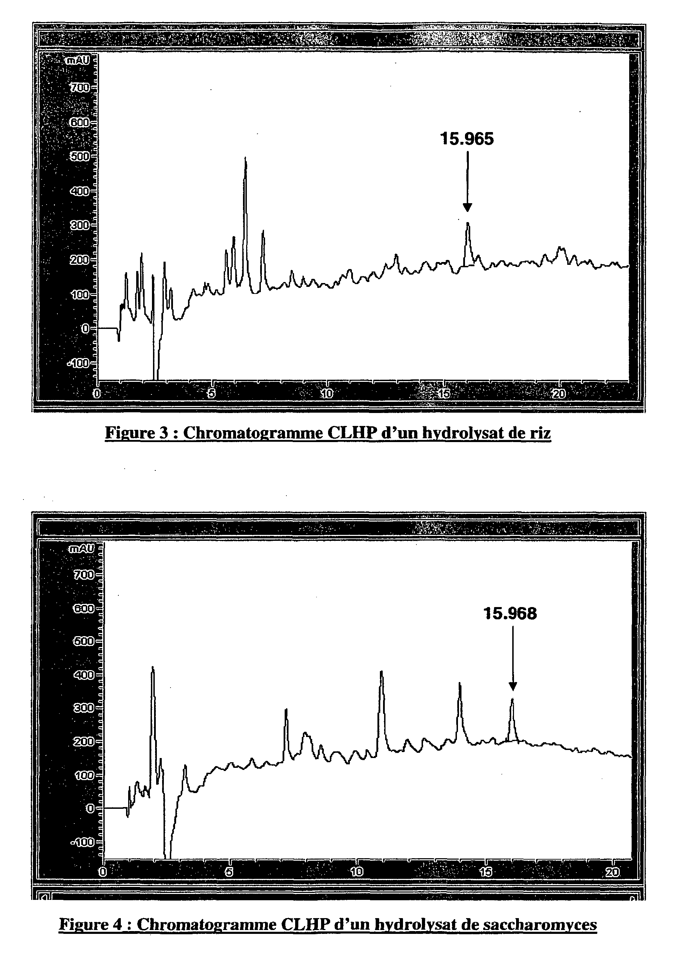

Ces diverses fractions sont ensuite analysées par spectrométrie de masse afin d'identifier spécifiquement le contenu en acides aminés des peptides de chaque pic. Il a également été réalisé une analyse par séquençage, pour déterminer la séquence peptidique des peptides bioactifs. Un exemple de chromatogramme obtenu par CLHP (chromatographie en phase liquide à haute pression), avec mise en évidence du pic correspondant aux peptides bioactifs, est donné à la figure 4.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 4 : Mise en évidence de l'effet dépigmentant d'un hydrolysat peptidique de levures enrichi en peptidesbioactifs, par des tests ex vivo

L'activité dépigmentante d'un hydrolysat de levures enrichi en peptides bioactifs réalisé selon l'invention, a été mise en évidence sur un échantillon de peau.

Des biopsies de 6 mm de diamètre sont prélevées sur des échantillons de peau humaine. Ces biopsies sont maintenues en survie *ex vivo* par culture en présence d'un milieu spécifique (DMEM 1 g/L, HAMF12, SVF et antibiotiques) sur des inserts déposés dans des plaques 6 puits. Les biopsies sont alors prétraitées pendant 24 heures avec l'hydrolysat peptidique à la concentration de 3 % à raison de 2 applications par jour. D'autres échantillons de peau ne seront pas prétraités avec ce même hydrolysat et serviront de condition contrôle. Par la suite, les biopsies sont soumises à des irradiations UVB à raison de 100 mJ/cm². Les biopsies prétraitées sont traitées à nouveau pendant 24 heures avec l'hydrolysat peptidique à la concentration de 3 % à raison de 2 applications par jour. Après ce second traitement, une évaluation quantitative de la quantité de mélanine présente dans l'épiderme des échantillons de peau est réalisée histologiquement, au microscope optique, après une coloration suivant la méthode de Fontana-Masson.

Pour cela, les biopsies de peau sont incluses dans la paraffine et des coupes histologiques de 4 µm d'épaisseur sont effectuées. Ces coupes sont alors colorées par la technique de Fontana-Masson : les lames sont déparaffinées, hydratées puis traitées avec une solution d'ammoniaque d'argent. Après un passage de deux minutes au micro-onde, les lames sont rincées, traitées avec du sodium thiosulfate, rincées à nouveau et contre-colorées avec de l'hématoxyline avant d'être déshydratées et montées sous lamelles permettant ainsi la visualisation au microscope optique de la mélanine présente dans l'épiderme.

### Résultats :

Avant irradiation UVB, les peaux non traitées présentent un niveau de pigmentation plus élevé comparé aux peaux traitées avec l'hydrolysat peptidique de levures. Après irradiation UVB, les peaux traitées avec ledit hydrolysat, présentent un niveau de pigmentation nettement inférieur comparé aux peaux non traitées de la condition contrôle. Par conséquent, ces résultats nous permettent de conclure qu'en l'absence d'une irradiation par les UVB, l'hydrolysat peptidique de levures enrichi en peptides bioactifs, diminue le taux de mélanine par comparaison avec l'échantillon de peau non traitée. Par ailleurs, la synthèse de mélanine, induite par les irradiations aux UVB, est très nettement atténuée lorsque les échantillons de peaux sont prétraités avec notre principe actif. Ainsi, l'hydrolysat peptidique selon l'invention permet de diminuer significativement la pigmentation de la peau et permet d'obtenir une inhibition de la synthèse de mélanine.

### Exemple 5 : Composition cosmétique dépigmentante avec filtre solaire

| ***Noms commerciaux*** | ***Noms INCI*** | ***% massique*** |
|---|---|---|
| ***PHASE A*** | | |
| Montanov 68 | Cetearyl Alcohol (and) Cetearyl Glucoside | 5.00 |
| Isopropyl Palmitate | Isopropyl Palmitate | 7.00 |
| Waglinol 250 | Cetearyl Ethylhexanoate | 3.00 |
| Dow Corning 200 | Dimethicone Polydimethylsiloxane | 0.50 |
| Parsol MCX | Ethylhexyl Methoxycinnamate | 3.00 |
| Parsol 1789 | Butyl Methoxydibenzoylmethane | 1.00 |
| Phenonip | Phenoxyethanol (and) Methylparaben (and) Ethylparaben (and) Butylparaben (and) Propylparaben (and) Isobutylparaben | 0.50 |
| Cegesoft PS6 | Vegetable Oil | 2.00 |
| Huile de Jojoba | Simmondsia Chinensis (Jojoba) Seed Oil | 5.00 |

| ***PHASE B*** | | |
|---|---|---|
| Eau Demineralisee | Aqua (Water) | qsp |
| Glycerine | Glycerin | 3.00 |
| Glucam E10 | Methyl Gluceth-10 | 0.50 |
| EDTA Tetrasodium | EDTA | 0.20 |

| ***PHASE C*** | | |
|---|---|---|
| Sepigel 305 | Polyacrylamide (and) C13-14 Isoparaffin (and) Laureth-7 | 0.35 |
| Jus de Citron | Citrus medica Limonum (Lemon) Fruit | 0.23 |

| ***Noms commerciaux*** | ***Noms INCI*** | ***% massique*** |
|---|---|---|
| | Extract | |
| ***PHASE D*** | | |
| Hydrolysat peptidique de riz | | 2.00 |
| Compo parfumante | Parfum (Fragrance) | qsp |
| Colorant | Dye | qsp |

## Revendications

1. Utilisation cosmétique d'une composition comprenant un hydrolysat peptidique issu de l'hydrolyse de levures du genre *Saccharomyces,* et plus particulièrement de l'espèce *Saccharomyces cerevisiae,* et enrichi en peptides bioactifs de poids moléculaire inférieur à 6 kDa, comportant de 3 à 5 acides aminés, chaque peptide bioactif comprenant au moins un résidu acide aspartique, un résidu cystéine et un résidu arginine et un milieu cosmétiquement acceptable, pour augmenter l'activité du protéasome et améliorer la dégradation par le protéasome des protéines endommagées impliquées dans la mélanogénèse.

2. Utilisation cosmétique selon la revendication 1, pour dépigmenter, éclaircir et/ou blanchir la peau.

3. Utilisation cosmétique selon la revendication 2, pour prévenir et/ou traiter les taches d'hyperpigmentation choisies parmi le mélasma, le chloasma, le lentigo actinique, les lentigos solaires, les éphélides, les taches d'hyperpigmentation accidentelles, les taches d'hyperpigmentation post-cicatricielles et les signes cutanés d'ordre hyperpigmentaire dus au photo-vieillissement.

4. Utilisation cosmétique selon l'une des revendications précédentes, **caractérisée en ce que** le peptide bioactif est de formule générale (I) :
X₁-[Asp, Cys, Arg] - X₂
dans laquelle,
X₁ est une asparagine, une lysine, un aspartate, une valine, une arginine, ou est absent,
X₂ est une histidine, une lysine, une arginine, ou est absent.

5. Utilisation cosmétique selon la revendication 4, **caractérisé en ce que** le peptide bioactif correspond à une des formules suivantes :
(SEQ ID n°1) Arg - Asp -Cys - Arg - Arg
(SEQ ID n°2) Asn - Asp -Cys - Arg - Lys
(SEQ ID n°3) Asp - Cys - Arg - His
(SEQ ID n°4) Val - Asp - Cys - Arg
(SEQ ID n°5) Asp - Cys - Arg.

6. Utilisation cosmétique selon l'une des revendications précédentes, **caractérisée en ce que** l'hydrolysat peptidique enrichi contient entre 0,5 et 5,5 g/l de composés de nature peptidique.

7. Utilisation cosmétique selon l'une des revendications précédentes, **caractérisée en ce que** la composition se présente sous une forme adaptée à l'application par voie topique.

8. Utilisation cosmétique selon l'une des revendications précédentes, **caractérisée en ce que** l'hydrolysat peptidique enrichi est présent dans la composition en une quantité représentant de 0,0001 % à 20 % du poids total de la composition.

9. Utilisation cosmétique selon la revendication 8, caractérisée en ce l'hydrolysat peptidique enrichi est présent dans la composition en une quantité représentant de 0,05 % à 5 % du poids total de la composition.

10. Utilisation cosmétique selon l'une des revendications précédentes, **caractérisée en ce que** la composition contient en outre, au moins un autre principe actif favorisant l'action dudit hydrolysat peptidique enrichi choisi parmi un agent présentant une activité dans le domaine des agents éclaircissants tels que des agents desquamants ; des agents apaisants, des agents photo-protecteurs organiques ou inorganiques, des agents hydratants ; d'autres agents dépigmentants, des inhibiteurs de tyrosinase ; des agents stimulant la synthèse de macromolécules dermiques ou épidermiques et/ou empêchant leur dégradation ; des agents stimulant la prolifération des fibroblastes et/ou des kératinocytes ; des agents agissant sur le métabolisme énergétique des cellules ; des peptides dépigmentants, des hydrolysats végétaux, des agents anti-âge, ainsi que leurs mélanges.

11. Procédé de traitement cosmétique **caractérisé en ce que** l'on applique topiquement sur la peau ou les phanères à traiter, une composition comprenant une quantité efficace d'un hydrolysat peptidique issu de l'hydrolyse de levures du genre *Saccharomyces,* et plus particulièrement de l'espèce *Saccharomyces cerevisiae,* et enrichi en peptides bioactifs de poids moléculaire inférieur à 6 kDa, comportant de 3 à 5 acides aminés, chaque peptide bioactif comprenant au moins un résidu acide aspartique, un résidu cystéine et un résidu arginine, et un milieu cosmétiquement acceptable, pour dépigmenter, éclaircir et/ou blanchir la peau ou les phanères.

## Patentansprüche

1. Kosmetische Verwendung einer Verbindung, umfassend ein Peptidhydrolysat, das aus der Hydrolyse von Hefen der Gattung *Saccharomyces* stammt, und insbesondere der Spezies *Saccharomyces cerevisiae,* und angereichert mit bioaktiven Peptiden mit einem Molekulargewicht von weniger als 6 kDa, umfassend 3 bis 5 Aminosäuren, wobei jedes bioaktive Peptid mindestens einen Asparaginsäure-Rückstand, einen Zystein-Rückstand und einen Arginin-Rückstand und ein kosmetisch akzeptables Milieu umfasst, um die Aktivität des Proteasoms zu erhöhen und den Abbau der beschädigten Proteine, die an der Melanogenose beteiligt sind, durch die Proteasome zu verbessern.

2. Kosmetische Verwendung nach Anspruch 1, um die Haut zu depigmentieren, zu bleichen und/oder aufzuhellen.

3. Kosmetische Verwendung nach Anspruch 2, um den Hyperpigmentierungsflecken vorzubeugen und/oder diese zu behandeln, ausgewählt aus dem Melasma, dem Chloasma, der aktinischen Lentigo, den Lentigos Solaris, den Sommersprossen, den zufälligen Hyperpigmentierungsflecken, den Hyperpigmentierungsflecken nach Vernarbungen und den Hautzeichen der Hyperpigmentierung aufgrund der Lichtalterung.

4. Kosmetische Verwendung nach einem der vorhergehenden Ansprüche, **dadurch gekennzeichnet, dass** das bioaktive Peptid der Folgenden allgemeinen Formel (I) ist:
X1 - [Asp, Cys, Arg] - X₂
wobei
X₁ ein Asparagin, ein Lysin, ein Aspartat, ein Valin, ein Arginin oder nicht vorhanden ist,
X₂ ein Histidin, ein Lysin, ein Arginin oder nicht vorhanden ist.

5. Kosmetische Verwendung nach Anspruch 4, **dadurch gekennzeichnet, dass** das bioaktive Peptid einer der folgenden Formeln entspricht :
(SEQ ID n°1) Arg - Asp -Cys - Arg - Arg
(SEQ ID n°2) Asn - Asp -Cys - Arg - Lys
(SEQ ID n°3) Asp - Cys - Arg - His
(SEQ ID n°4) Val - Asp - Cys - Arg
(SEQ ID n°5) Asp - Cys - Arg.

6. Kosmetische Verwendung nach einem der vorhergehenden Ansprüche, **dadurch gekennzeichnet, dass** das angereicherte Peptidhydrolysat zwischen 0,5 und 5,5 g/l Verbindungen peptidischer Art umfasst.

7. Kosmetische Verwendung nach einem der vorhergehenden Ansprüche, **dadurch gekennzeichnet, dass** die Verbindung eine Form aufweist, die für die Anwendung auf topischem Weg ausgelegt ist.

8. Kosmetische Verwendung nach einem der vorhergehenden Ansprüche, **dadurch gekennzeichnet, dass** das angereicherte Peptidhydrolysat in der Verbindung in einer Menge vorhanden ist, die von 0,0001 % bis 20 % des Gesamtgewichts der Verbindung darstellt.

9. Kosmetische Verwendung nach Anspruch 8, **dadurch gekennzeichnet, dass** das angereicherte Peptidhydrolysat in der Verbindung in einer Menge vorhanden ist, die von 0,05 % bis 5 % des Gesamtgewichts der Verbindung darstellt.

10. Kosmetische Verwendung nach einem der vorhergehenden Ansprüche, **dadurch gekennzeichnet, dass** die Verbindung außerdem mindestens einen weiteren Wirkstoff umfasst, der die Wirkung des angereicherten Peptidhydrolysats fördert, ausgewählt aus einem Mittel, das eine Aktivität auf dem Gebiet der bleichenden Mittel umfasst wie z.B. abschuppende Mittel, lindernd wirkende Mittel, organische oder anorganische Lichtschutzmittel, Befeuchtungsmittel; andere Depigmentierungsmittel, Tyrosinasehemmer, Mittel, die die Synthese von Haut- oder Oberhautmakromolekülen stimulieren und/oder ihren Abbau verhindern; Mittel, die die Proliferation der Fibroblasten und/oder der Keratinozyten stimulieren; Mittel, die auf den Energiestoffwechsel der Zellen wirken; Depigmentierungspeptide, pflanzliche Hydrolysate, Anti-Aging-Mittel; ebenso wie ihre Mischungen.

11. Verfahren zur kosmetischen Behandlung, **dadurch gekennzeichnet, dass** topisch auf die zu behandelnde(n) Haut/Hautanhangsgebilde eine Verbindung aufgebracht wird, die eine wirksame Menge eines Peptidhydrolysats umfasst, das aus der Hydrolyse von Hefen der Gattung *Saccharomyces* stammt, und insbesondere der Spezies *Saccharomyces cerevisiae,* und angereichert mit bioaktiven Peptiden mit einem Molekulargewicht von weniger als 6 kDa, umfassend von 3 bis 5 Aminosäuren, wobei jedes bioaktive Peptid mindestens einen Asparaginsäure-Rückstand, einen Zystein-Rückstand und einen Arginin-Rückstand und ein kosmetisch akzeptables Milieu umfasst, um die Haut oder die Hautanhangsgebilde zu depigmentieren, zu bleichen und/oder aufzuhellen.

## Claims

1. Cosmetic use of a composition comprising a peptide hydrolysate obtained from the hydrolysis of yeasts of the *Saccharomyces* genus, and more particularly the species *Saccharomyces cerevisiae,* and enriched with bioactive peptides having a molecular weight less than 6 kDa, comprising 3 to 5 amino acids, each bioactive peptide comprising at least one aspartic acid residue, one cysteine residue and one arginine residue and a cosmetically acceptable medium, for increasing the proteasome activity and enhancing the degradation by proteasome of damaged proteins involved in melanogenesis.

2. Cosmetic use according to claim 1, for depigmenting, lightening and/or whitening skin.

3. Cosmetic use according to claim 2, for preventing and/or treating hyperpigmentation spots chosen from melasma, chloasma, actinic lentigo, solar lentigenes, ephelides, accidental hyperpigmentation spots, post-scarring hyperpigmentation spots and hyperpigmentary skin signs due to photo-ageing.

4. Cosmetic use according to any of the above claims, **characterised in that** the bioactive peptide has the following general formula (I):
X₁ - [Asp, Cys, Arg] - X₂
wherein,
X₁ is an asparagine, a lysine, an aspartate, a valine, an arginine, or is absent,
X₂ is a histidine, a lysine, an arginine, or is absent.

5. Cosmetic use according to claim 4, **characterised in that** the bioactive peptide corresponds to one of the following formulas:
(SEQ ID No. 1) Arg - Asp - Cys - Arg - Arg
(SEQ ID No. 2) Asn - Asp - Cys - Arg - Lys
(SEQ ID No. 3) Asp - Cys - Arg - His
(SEQ ID No. 4) Val - Asp - Cys - Arg
(SEQ ID No. 5) Asp - Cys - Arg.

6. Cosmetic use according to any of the above claims, **characterised in that** the enriched peptide hydrolysate contains between 0.5 and 5.5 g/l of peptide type compounds.

7. Cosmetic use according to any of the above claims, **characterised in that** the composition is presented in a form suitable for topical application.

8. Cosmetic use according to any of the above claims, **characterised in that** the enriched peptide hydrolysate is present in the composition in a quantity representing from 0.0001% to 20% of the total weight of the composition.

9. Cosmetic use according to claim 8, **characterised in that** the enriched peptide hydrolysate is present in the composition in a quantity representing from 0.05% to 5% of the total weight of the composition.

10. Cosmetic use according to any of the above claims, **characterised in that** the composition further contains at least one further active substance promoting the action of said enriched peptide hydrolysate chosen from an agent having an activity in the field of lightening agents such as desquamative agents; soothing agents, organic or inorganic photo-protective agents, moisturising agents; further depigmenting agents, tyrosinase inhibitors; agents stimulating dermal or epidermal macromolecule synthesis and/or preventing the degradation thereof; agents stimulating fibroblast and/or keratinocyte proliferation; agents acting on the cell energy metabolism; depigmenting peptides, plant hydrolysates, anti-age agents, and mixtures thereof.

11. Cosmetic treatment method **characterised in that** a composition comprising an effective quantity of a peptide hydrolysate obtained from the hydrolysis of yeasts of the *Saccharomyces* genus, and more particularly the species *Saccharomyces cerevisiae,* and enriched with bioactive peptides having a molecular weight less than 6 kDa, comprising 3 to 5 amino acids, each bioactive peptide comprising at least one aspartic acid residue, one cysteine residue and one arginine residue and a cosmetically acceptable medium is applied topically onto the skin or skin appendages to be treated, for depigmenting, lightening and/or whitening skin or skin appendages.
